Europäisches Patentamt

European Patent Office

Office européen des brevets

(11) Publication number: **0 419 292 A1**

## EUROPEAN PATENT APPLICATION

(21) Application number: 90310395.0

(22) Date of filing: 21.09.90

(51) Int. Cl.⁵ **C12N 9/76**, C12P 21/08, A61K 39/395, A61K 37/547

(30) Priority: 22.09.89 JP 247893/89

(43) Date of publication of application:
27.03.91 Bulletin 91/13

(84) Designated Contracting States:
AT BE CH DE DK ES FR GB GR IT LI LU NL SE

(71) Applicant: TAISHO PHARMACEUTICAL CO. LTD
24-1 Takata 3-chome Toshima-ku
Tokyo 171(JP)

(72) Inventor: Katunuma, Nobuhiko
246-2, Myodocho-3-chome
Tokushima-shi(JP)
Inventor: Yoshimoto, Makoto
Nishiageo Daiichidanchi 1-23-302, 845-1,
Koshikiya
Ageo-shi(JP)

(74) Representative: Lamb, John Baxter et al
MARKS & CLERK 57/60 Lincoln's Inn Fields
London WC2A 3LS(GB)

(54) Human tryptase-like protein.

(57) Novel human tryptase-like protein having both trypsin-like and chymotrypsin-like activity specifically binds with anti-rat tryptase antibody and comprises two subunits of about 35±3 KDa and four subunits of about 30±2 KDa. The human tryptase-like protein can be obtained from human acute lymphoblastic leukemia cell line, Molt 4. The anti-human tryptase-like protein antibody strongly inhibits HIV infection, which is indicated by a potent inhibition of syncytia formation. Pharmaceutical compositions comprising the anti-human tryptase-like protein antibody are effective for the treatment of AIDS.

EP 0 419 292 A1

# HUMAN TRYPTASE-LIKE PROTEIN

The present invention relates to a novel human tryptase-like protein which can be purified from human T cells, anti-human tryptase-like protein antibody which can bind specifically to the human tryptase-like protein, a composition for the treatment of acquired immunodeficiency syndrome (AIDS) comprising the anti-human tryptase-like protein antibody as an effective ingredient and a vaccine for acquired immunodeficiency syndrome (AIDS) comprising the human tryptase-like protein.

Acquired immunodeficiency syndrome (AIDS) is a serious immunodeficiency induced by human immunodeficiency virus (HIV) and is now spreading worldwide. It has been desired to develop a drug for the treatment of AIDS or a vaccine thereof.

It was revealed that HIV binds to helper T cells, which play the central role in immunoreaction, and invade into the cell to kill helper T cells, and this causes immunodeficiency. It has also been revealed that HIV infection is initiated by the binding of gp120, an envelope glycoprotein of HIV, to CD4 molecules on the surface of helper T cells [Dalgleis et al., Nature, 312 , 763 (1984); Klatzmann et al., Nature, 312 , 767 (1984); McDougal et al., Science, 231 , 382 (1986)].

Based upon such mechanism of infection, an approach has been made using antibodies to gp120 as a drug to inhibit HIV infection.

It was found by the present inventor that trypstatin, which is a Kunitz type protease inhibitor, isolated from rat mast cells, had a high homology to the variable region of gp120 and trypstatin could thus be used as an inhibitor of HIV infection [Japanese Patent Application No. 1-2579; FEBS LETTERS, Vol. 248, No. 1, 2, pages 48-52 (1989)].

It was also revealed by the present inventors that antibody to tryptase, one of serine proteases, which was purified from rat tongue mast cells and inhibited by trypstatin, inhibited HIV infection. [Japanese Patent Application No. 1-6985; FEBS LETTERS, Vol. 248, No. 1, 2, pages 48-52 (1989)].

These facts may also suggest that tryptase-like proteins which can be inhibited by trypstatin and anti-tryptase antibody are present on the surface of HIV-sensitive human T cells. However, there is no report so far on isolation and purification of such a protein.

## SUMMARY OF THE INVENTION

An object of the present invention is to provide a novel human tryptase-like protein present in human T cells.

Another object of the present invention is to provide an anti-human tryptase-like protein antibody capable of specifically reacting with the human tryptase-like protein present in human T cells.

A further object of the present invention is to provide a composition for the treatment of AIDS comprising the anti-human tryptase-like protein antibody as an effective ingredient.

A still further object of the present invention is to provide a vaccine for AIDS comprising the human tryptase-like protein.

Under the concept that tryptase-like protein would be present on the surface of human T cells and this protein would play an important role as a receptor of HIV, the present inventor has made investigations and has come to accomplish the present invention.

That is, the present inventors have succeeded in purifying a novel tryptase-like protein, which can bind to anti-rat tryptase antibody, from human acute lymphoblastic leukemia cells, Molt 4. It has thus been found that an antibody to this protein can strongly inhibit HIV infection and based on this finding, the present invention has been attained.

In one aspect, the present invention is directed to a human tryptase-like protein comprising 2 subunits of about 35±3 KDa and 4 subunits of about 30±2 KDa, which can bind to anti-rat tryptase antibody and can be obtained from human T cells.

In another aspect, the present invention is directed to an anti-human tryptase-like protein antibody or a fragment of the antibody which can specifically bind to the human tryptase-like protein or its antigenic binding site.

In a further aspect, the present invention is directed to a composition for the treatment of AIDS comprising the anti-human tryptase-like protein antibody or a fragment thereof containing the antigen binding site as an effective ingredient.

In a still further aspect, the present invention is directed to a vaccine for AIDS comprising the human tryptase-like protein.

BRIEF DESCRIPTION OF THE DRAWINGS

Fig. 1 shows results obtained when human tryptase-like protein is subjected to 1% agarose gel electrophoresis.

Fig. 2 shows results when human tryptase-like protein is subjected to SDS-polyacrylamide gel electrophoresis under reducing conditions and results of Western blot analysis.

Fig. 3 is a graph showing the optimum pH of human tryptase-like protein.

DETAILED DESCRIPTION OF THE PREFERRED EMBODIMENTS

The human tryptase-like protein of the present invention can be isolated and purified from human T cells as described below.

As human T cells, for example, human acute lymphoblastic leukemia cells, Molt 4 [ATCC No. CRL-1582] can be used. The cultured cells are homogenized and centrifuged. The resulting supernatant is used as the extract. After acid precipitation and centrifugation, polyethylene glycol is added to the resulting supernatant. Then the mixture is centrifuged to obtain precipitate. This precipitate is used as a sample for the subsequent purification by chromatography.

The obtained sample is applied to high performance liquid chromatography using an ion exchange column, such as DEAE-3SW column, etc. Fractions which contain human tryptase-like protein are determined by measuring trypsin activity using Boc-Gln-Gly-Arg-MCA as substrate, and reactivity with anti-rat tryptase antibody. The active fractions are collected and subjected to hydroxyappatite column chromatography and then gel filtration column chromatography. Thus, the human tryptase-like protein of the present invention can be isolated and purified.

The human tryptase-like protein of the present invention has the following properties.

(i) Molecular weight

The molecular weight determined by gel filtration is about 198 KDa.

(ii) Construction

When subjected to 1% agarose gel electrophoresis, a single band can be detected. In SDS-polyacrylamide gel electrophoresis under reducing conditions, two bands of about 35±3 KDa and about 30±2 KDa can be detected. Intensity ratio of these bands by silver staining is 1 : 2. From these facts and the molecular weight, the human tryptase-like protein of the present invention is a complex composed of 2 subunits of about 35±3 KDa and 4 subunits of about 30±2 KDa.

(iii) Reactivity with antibody

The protein binds with an antibody to tryptase purified from rat tongue mast cells. However, only the subunits of about 35±3 KDa bind with this antibody and the subunits of about 30±2 KDa do not bind therewith. These facts reveal that the protein of the present invention is a tryptase-like protein.

(iv) Enzyme activity

The protein cleaves Boc-Gln-Gly-Arg-MCA, a substrate for trypsin, and Suc-Leu-Leu-Val-Tyr-MCA, a substrate for chymotrypsin. It is thus shown that the human tryptase-like protein of the present invention has both trypsin-like activity and chymotrypsin-like activity.

Trypsin-like activity of the human tryptase-like protein of the present invention is inhibited by trypstatin, leupeptin, antipain and chymostatin. However, the protein of the present invention is little affected by other inhibitors against proteases such as cystein protease, amino-peptidase, metal protease, acidic protease, etc. Therefore, it is considered that the human tryptase-like protein of the present invention would be a sort of serine protease.

(v) Optimum pH

The human tryptase-like protein of the present invention shows the most potent trypsin-like, and chymotrypsin-like activity at pH 8.5.

(vi) Stability

Storage of the human tryptase-like protein in 25 mM ammonium formate (pH 5.5)/50% glycerol solution at -20°C for one month decreses the activity to about 1/4. At room temperature, the activity is completely lost within one hour.

(vii) Antibody productivity

By injecting the human tryptase-like protein of the present invention to animal, production of anti-human tryptase-like protein antibody is induced. This antibody can prevent syncytium formation induced by HIV infection.

As stated above in detail, the human tryptase-like protein of the present invention which can be perified from human T cells is a new type protein having various characteristic properties.

The human tryptase-like protein of the present invention can induce production of the anti-human tryptase-like protein antibody having an ability of suppressing HIV infection, as described above. The anti-human tryptase-like protein antibody can be used as an agent for the treatment of AIDS, because the antibody has an ability of suppressing HIV infection.

The anti-human tryptase-like protein antibody of the present invention can be obtained by immunizing animals with human tryptase-like protein and preparing sera from the animals. The anti-human tryptase-like protein antibody of the present invention can also be obtained by immunizing animals with the subunits of about 35±3 KDa or subunits of about 30±2 KDa of the human tryptase-like protein.

Any animal can be immunized with human tryptase-like protein, eg., rabbits, mice, sheep, horses, etc. For immunization, human tryptase-like protein is mixed with Freund's complete adjuvant and administered instractaneously several times in a definite time interval. After immunization, sera are prepared and anti-human tryptase-like protein IgG fraction can be obtained by, for example, fractionation with ammonium sulfate, DE52 column chromatography, etc. Alternatively, the IgG fraction may be obtained by preparing the gamma globulin-containing fraction from sera by the ethanol fractionation [Cohn et al., J. Am. Chem. Soc., 68 , 459 (1946)] and then precipitating with polyethylene glycol, ethanol, etc. [Schultze et al., "Molecular Biology of Human Proteins", Elsevier, Amsterdam, 2 , 256 (1966)].

The anti-human tryptase-like protein antibody of the present invention may be a monoclonal antibody. Such monoclonal antibody can be prepared in a conventional manner known as the method of Kohler and Milstein [Kohler et al., Nature, 256 , 495 (1975)]. That is, animal is immunized with human tryptase-like protein and then spleen cells are prepared from the immunized animal. The spleen cells are fused with myeloma cells to obtain hybridoma. By culturing the hybridoma in vivo or in vitro , the monoclonal antibody can be obtained.

In order to further reduce the antigenecity of the monoclonal antibody, chimera antibody may also be used which is prepared by known methods in which the variable region from mouse antibody and the constant region from human antibody are fused [Sahagan et al., J. Immunology, 137 , 1066 (1986); Nishimura et al., Cancer Research, 47 , 999 (1987); Riechmann et al., Nature, 332 , 323 (1988)].

The anti-human tryptase-like protein antibody of the present invention may be a fragment containing its antigen binding site. The antibody may also be used in the form of F(ab')₂ fragment obtained by digesting the antibody with, e.g., pepsin [Nisonofff et al., Arch. Biochem. Biophys., 89 , 230 (1960); Ishizuka et al., J. Immunology, 120 , 800 (1978)] or in the form of Fab fragment obtained by digesting the antibody with papain [R.R. Porter, Biochemical Journal, 73 , 119 (1959)].

It has been clarified by the present inventor that the anti-human tryptase-like protein antibody or its fragment can strongly inhibit HIV infection. That is, when MOLT-4 cells (ATCC Number: CRL-1582) which are human acute lymphoblastic leukemia cells and CEM/LAV-1 cells obtained by persistent infection of CCRF-CEM cells (ATCC CCL-119) with LAV-1 (one of HIV) [Barre-Sinoussi et al., Science, 220 , 868 (1983)] are co-cultured, syncytia are formed. In the case that the anti-human tryptase-like protein antibody or its fragment is added to the system, it has been confirmed that the formation of syncytia is inhibited and hence, the anti-human tryptase-like protein antibody or its fragment can inhibit HIV infection.

4

Therefore, the anti-human tryptase-like protein antibody of the present invention or its fragment is extremely useful as drugs for the treatment of AIDS.

The anti-human tryptase-like protein antibody or its fragment may be generally administered to patients with AIDS parenterally, e.g., intravenously, intramuscularly, subcutaneously, intrarectally, etc. An example of parenteral preparations is an injection. The injection may be prepared in a conventional manner, for example, by subjecting the IgG fraction obtained as described above or its fragment to dialysis or sterile filtration, etc., if necessary and desired, and then dissolving or dispersing in an aqueous medium. In order to purify further, the IgG fraction or its fragment may also be further purified using an affinity column, onto which human tryptase-like protein has been adsorbed. The injections may contain mannitol, lactose, albumin, etc. In addition, preservatives, stabilizers, antiseptics, surface active agents which are conventionally used may also be incorporated into the injections, if necessary and desired.

A dose of the anti-human tryptase-like protein antibody or its fragment may vary depending upon age, body weight or conditions of patients, etc. but may be generally in the range of from 20 to 2,000 mg/kg-body weight/day, preferably 50 to 1,000 mg/kg-body weight/day.

As is clear from the foregoing detailed description, the anti-human tryptase-like protein antibody can be produced in vivo by immunizing animal with the human tryptase-like protein and the anti-human tryptase-like protein antibody strongly supress HIV infection. From such facts, it will be apparent to one skilled in the art that the human tryptase-like protein itself can be used as a peptide vaccine for AIDS.

In the vaccine preparations of human tryptase-like protein, the human tryptase-like protein per se , or denatured or modified human tryptase-like protein may be used solely or in combination with adjuvant. Examples of such adjuvant include aluminum hydroxide, aluminum phosphate, calcium phosphate or derivatives of muramyl dipeptide which is a constituent of the cell wall of tubercle bacillus, and the like.

Human tryptase-like protein as the vaccine may be generally administered parenterally, e.g., intravenously, intramuscularly, subcutaneously, intrarectally, etc., as in the case of the anti-human tryptase-like protein antibody. A dose is in the range of from 0.2 to 20 μg/kg body weight, preferably 1 to 10 μg/kg body weight, per administrtion, as human tryptase-like protein.

The novel human tryptase-like protein which bind with anti-rat tryptase antibody can be isolated from human T cells.

The anti-human tryptase-like protein antibody which can specifically bind to the human tryptase-like protein has a strong activity to suppress HIV infection and is thus extremely effective as a drug for the treatment of AIDS. The human tryptase-like protein can be used as a vaccine for AIDS since the human tryptase-like protein induces production of the anti-human tryptase-like protein antibody in vivo and the anti-human tryptase-like protein antibody strongly suppresses HIV infection.

The present invention will be described in more detail by referring to test examples and examples but is not deemed to be limited thereto.

Example 1

Isolation and purification of human tryptase-like protein and its properties

I. Culture of human lymphoblastic leukemia cells, Molt 4 and purification of human tryptase-like protein from the cells

Human lymphoblastic leukemia cells, Molt 4, was cultured to $10^6$ cells/ml in 25 liters of RPMI 1640 medium (manufactured by GIBCO Co.). Cells were harvested by centrifugation (750g, 10 minutes), and about 18 g of cells (wet weight) were obtained. To the cells was added 90 ml of 20 mM Tris-HCl (pH 7.0), and the cells were homogenized and then centrifuged (20,000g, 15 minutes) to obtain the supernatant. The extraction was repeated twice and the supernatants were combined. After adjusting pH to 5.5 with acetic acid, 1 M sodium acetate (pH 5.5) was added to the final concentration of 50 mM. After centrifugation (20,000 g, minutes), polyethylene glycol (PEG-6000) was added to the supernatant to the final concentration of 2%. The mixture was allowed to stand on ice for 30 minutes. After centrifugation (20,000 g, 10 minutes), PEG-6000 was further added to the supernatant to the final concentration of 10%. After stirring on ice for 30 minutes, the precipitates were collected by centrifugation (20,000 g, 15 minutes). The precipitates were dissolved in a small volume of 25 mM Tris-HCl (pH 6.5). This solution was further purified by chromatography.

2. Purification by chromatography

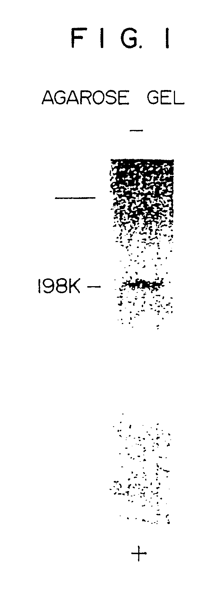

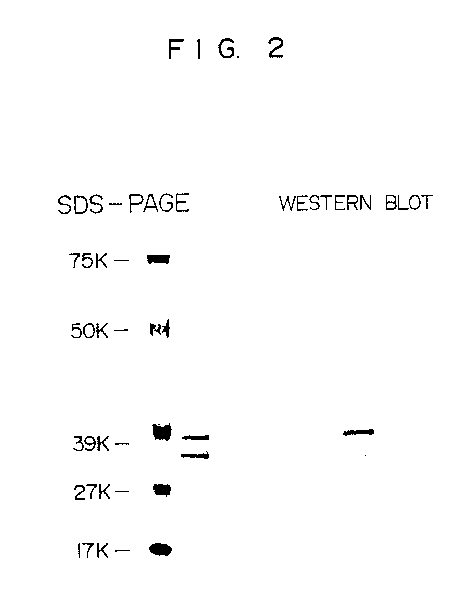

The sample obtained in 1. was purified by high performance liquid chromatography (HPLC) using ion exchange column, DEAE 3SW (7.5 mm x 7.5 cm). The human tryptase-like protein was eluted with a linear gradient of 25 mM to 1 M Tris-HCl buffer (1 ml/min/tube). Active fractions which were determined by measuring trypsin activity using Boc-Gln-Gly-Arg-MCA as a substrate, and binding activity with anti-rat tongue mast cell tryptase antibody [FEBS LETTERS, Vol. 248, No. 1, 2, pages 48-52 (1989)] were collected. The fractions were concentrated to a small volume by ultrafiltration on a YM-10 membrane (manufactured by Amicon Co., Ltd.). The concentrated sample was applied to hydroxyappatite column (HCA-Column A-7610, 7.6 mm x 10 cm), which was eluted with linear gradient of 10 mM to 0.8 M potassium phosphate buffer (1 ml/min/tube). Active fractions were collected and concentrated to a small volume by ultrafiltration. Furthermore, the concentrated sample was fractionated through HPLC gel filtration columns, G3000 SW (manufactured by Toso Co., Ltd., 7.5 mm x 60 cm, 2 columns), equilibrated with 25 mM ammonium formate (pH 5.5)/1 M urea (0.45 ml/min/tube). Active fractions were collected and concentrated to a small volume by ultrafiltration. When the sample was fractionated by 1% agarose gel electrophoresis, a single band was detected (cf. Fig. 1), and two bands of about 35±3 KDa and about 30±2 KDa were detected by SDS-polyacrylamide gel electro phoresis under reducing conditions (cf. Fig. 2). The intensity of protein stained by silver staining revealed that the subunits of about 35±3 KDa and about 30±2 KDa were present in a ratio of 1 : 2. On the other hand, from the elution pattern of gel filtration, the molecular weight of human tryptase-like protein was estimated to be about 198 KDa. It was thus shown that the human tryptase-like protein was a complex composed of two subunits of about 35±3 KDa and four subunits of about 30±2 KDa. Among the subunits, only the subunits of about 35±3 KDa bound with anti-rat tongue mast cell tryptase antibody, and the subunits of about 30±2 KDa did not bind therewith. That is, when tryptase-like protein which was fractionated by SDS-polyacrylamide gel electrophoresis was transferred onto a membrane filter and exposed to anti-rat tryptase antibody (Western blot analysis), only the subunits of about 35±3 KDa bound with the antibody (cf. Fig. 2).

3. Measurement of enzyme activity and effects of additives on the activity

Enzyme activity of the human tryptase-like protein during the course of purification or after purification was measured as follows. The enzyme sample was added to 0.1 M Tris-HCl buffer (pH 7.8) containing 25 mM calcium chloride and 100 $\mu$M of fluorogenic substrate. The fluorescence was measured with excitation and emmision wavelength of 380 nm and 460 nm, respectively. Effects of inhibitors on the enzyme activity were examined by adding 10 $\mu$M each of inhibitor to the reaction solution described above.

Substrate specificity of human tryptase-like protein are shown in Table 1.

EP 0 419 292 A1

Table 1.

| Substrate Specificity | | |
|---|---|---|
| Substrate (100 $\mu$M) | Activity ($\mu$U/ml) | % |
| Trypsin-type: | | |
| Boc-Gln-Gly-Arg-MCA | 754.4 | 100 |
| Boc-Gln-Ala-Arg-MCA | 532.4 | 71 |
| Boc-Ile-Glu-Gly-Arg-MCA | 355.0 | 47 |
| Glt-Gly-Arg-MCA | 332.8 | 44 |
| Boc-Glu-Lys-Lys-MCA | 221.9 | 29 |
| Boc-Ala-Gly-Pro-Arg-MCA | 199.7 | 26 |
| Boc-Gln-Arg-Arg-MCA | 177.5 | 24 |
| Boc-Gly-Lys-Arg-MCA | 177.5 | 24 |
| Boc-Phe-Ser-Arg-MCA | 177.5 | 24 |
| Pro-Phe-Arg-MCA | 155.3 | 21 |
| Boc-Leu-Arg-Arg-MCA | 110.9 | 15 |
| Boc-Leu-Thr-Arg-MCA | 110.9 | 15 |
| Boc-Val-Pro-Arg-MCA | 66.6 | 9 |
| Boc-Gly-Arg-Arg-MCA | 44.4 | 6 |
| Chymotrypsin-type: | | |
| Suc-Leu-Leu-Val-Tyr-MCA | 244.1 | 32 |
| Suc-Ala-Ala-Pro-Phe-MCA | 110.9 | 15 |
| Boc : N-t-butoxycarbonyl<br>MCA : 4-methylcoumaryl-7-amide<br>Suc : succinyl<br>Glt : glutaryl | | |

As is clear from Table 1, among the substrates examined, Boc-Gln-Gly-Arg-MCA (manufactured by Peptide Research Institute) developed as a substrate for Factor XIIa was hydrolyzed most rapidly by the trypsin-like activity of the human tryptase-like protein. On the other hand, Suc-Leu-Leu-Val-Tyr-MCA (manufactured by Peptide Research Institute) was hydrolized most rapidly by the chymotrypsin-like activity of the protein.

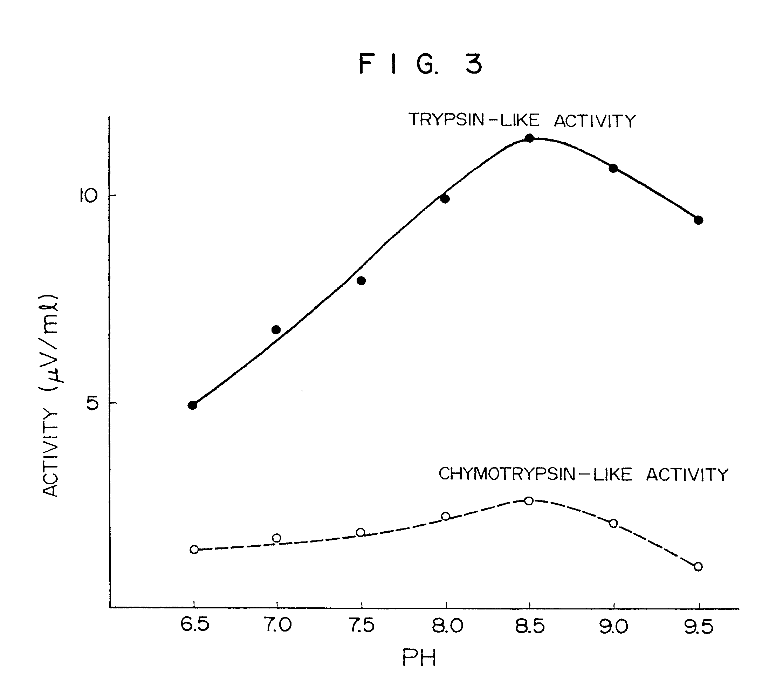

Using these substrates, the pH dependency of enzyme activity was examined. The optimum pH of both trypsin-like and chymotrypsin-like activity were 8.5 (Fig. 3).

Effects of inhibitors on the trypsin-like activity of the human tryptase-like protein was examined using Boc-Gln-Gly-Arg-MCA as substrate. The results are shown in Table 2.

7

Table 2.

| Influence of Inhibitor | | |
|---|---|---|
| Inhibitor | Activity (μU/ml) | Inhibition (%) |
| - | 843.1 | 0 |
| Trypstatin | 67.4 | 92 |
| Leupeptin | 110.9 | 87 |
| Antipain | 44.4 | 95 |
| HIαTI (human inter α-trypsin inhibitor) | 665.6 | 21 |
| A4-inhibitor | 682.9 | 19 |
| Aprotinin | 621.3 | 26 |
| αI-Antitrypsin | 687.8 | 18 |
| TLCK (Nα-p-tosyl-L-lysine chloromethyl ketone) | 691.3 | 18 |
| PMSF (phenylmethylsulfonyl fluoride) | 732.2 | 13 |
| Benzamidine | 817.8 | 3 |
| Chymostatin | 443.8 | 47 |
| BBI (Bowman-Birk inhibitor) | 665.6 | 21 |
| TPCK (N-tosyl-L-phenylalanine chloromethyl ketone) | 708.2 | 16 |
| αI-Antichymotrypsin | 845.1 | 0 |
| Elastatinal | 710.0 | 16 |
| Amastatin | 767.2 | 9 |
| Pepstatin | 845.0 | 0 |
| Bestatin | 843.0 | 0 |
| E-64c | 754.4 | 11 |
| Phosphoramidone | 820.9 | 3 |

As is shown in Table 2, the trypsin-like activity of the human tryptase-like protein was strongly inhibited by 10 μM each of trypstatin, leupeptin and antipain, but was not so strongly inhibited by other trypsin inhibitors. The trypsin-like activity was also inhibited by chymostatin, a chymotrypsin inhibitor. On the other hand, the trypsin-like activity was little inhibited by inhibitors of cystein proteases, aminopeptidases, metal proteases, acidic proteases, etc. Stability of the human tryptase-like protein was examined, and it was revealed that when the protein was stored at -20° C for a month in 25 mM ammonium formate (pH 5.5)/50% glycerol solution, the activity was reduced to about 1/4, and at room temperature, the activity was completely lost within one hour.

Example 2

Preparation of anti-human tryptase-like protein antibody

1. Preparation of anti sera

Human tryptase-like protein (100 μg, 0.1 ml) obtained by the method described above was thoroughly mixed with 0.5 ml of Freund's complete adjuvant and 0.15 ml each of the mixture was administered intradermally to four pads of a rabbit. Three weeks later, 60 μg of the human tryptase-like protein was administered to the same rabbit in a similar manner. After further 3 weeks, 60 μg of the human tryptase-like protein was administered to the same rabbit in a similar manner. Blood was collected four days after the final immunization and sera were prepared.

2. Preparation of anti-human tryptase-like protein IgG

To the rabbit anti-human tryptase-like protein sera obtained in 1. was added 4-fold amount of PBS (50 mM phosphate buffer (pH 7.2) containing 0.1 M sodium chloride). An equal volume of 36% sodium sulfate was added to the mixture and stirred at room temperature for 30 minutes. After centrifugation at 12,000 x g for 20 minutes, the collected precipitates were suspended in a small volume of 50 mM phosphate buffer (pH 7.6) containing 0.15 M sodium chloride. The suspension was passed through ULTROGEL AcA34 (manufactured by LKB Co., Ltd.). Eluted fractions containing IgG were collected and dialyzed against 10 mM Tris-HCl (pH 8.0) at 4°C overnight. Then the sample was subjected to DE52 column (manufactured by Whatman Co., Ltd.) previously equilibrated with 10 mM Tris-HCl (pH 8.0). After thoroughly washing with the same buffer, IgG was eluted with a linear gradient of 0 mM to 100 mM NaCl in 10 mM Tris-HCl (pH 8.0). IgG-containing fractions were collected and concentrated by ultrafiltration. It was confirmed that IgG was contained in this fraction by SDS-polyacrylamide gel electrophoresis [Laemmli, Nature, 227 , 680 (1970)].

3. Property of anti-human tryptase-like protein IgG

(1) Confirmation of reactivity of anti-human tryptase- like protein IgG with human tryptase-like protein by Ouchterlony's method

It was confirmed by the Ouchterlony's method that the purified anti-human tryptase-like protein IgG obtained in 2. bound with the human tryptase-like protein [Ouchterlony, Progr. Allergy, VI, 30 (1962)]. Agarose was dissolved in PBS at a concentration of 1% (w·v) and poured on a glass plate. Then, wells were formed with a puncher and the antibody and the purified human tryptase-like protein were dispensed therein. After several hours at room temperature, sedimentation line could be observed between the anti-human tryptase-like protein IgG and the purified human tryptase-like protein. No sedimentation line could be observed between the IgG purified from non-immunized rabbit sera and the purified human tryptase-like protein.

(2) Confirmation of reactivity of anti-human tryptase-like protein IgG with human tryptase-like protein by Western blotting method

It was also confirmed by the Western blotting method that the purified anti-human tryptase-like protein IgG obtained in 2. bound with the human tryptase-like protein [Towbin et al., Proc. Natl. Acad. Sci. USA, 76 , 4350 (1979)]. The purified human tryptase-like protein was fractionated by SDS-polyacrylamide gel electrophoresis and transferred electrophoretically onto a nitro cellulose filter in 25 mM Tris-HCl (pH 8.3)-192 mM glycine/20% methanol. After blocking with 10% fetal calf serum (FCS) in TBS (50 mM Tris-HCl (pH 8.0)/150 mM sodium chloride) at room temperature overnight, the filter was treated with anti-human tryptase-like protein IgG diluted with TBS/1% bovine serum albumin at room temperature for 5 hours. After washing 5 times with TBS/0.05% Tween 20 at room temperature for 5 minutes, a band of antibody-bound protein was detected by Pico Blue Immunodetection Kit (manufactured by STRATAGENE Co., Ltd). The results revealed that the anti-human tryptase-like protein IgG bound with the purified human tryptase-like protein.

4. Preparation of F(ab')$_2$ from the anti-human tryptase-like protein antibody

The anti-human tryptase-like protein IgG was cleaved with pepsin to prepare F(ab')$_2$ [Nisonofff et al., Arch. Biochem. Biophys., 89 , 230 (1960); Mandy et al., J. Biol. Chem., 238 , 206 (1963)]. To the anti-human tryptase-like protein IgG was added 1.50 amount of pepsin. The mixture was incubated in 0.1 M acetate buffer (pH 4.5) at 37°C for 18 hours. After the pH was adjusted to 8.0 by dropwise addition of 1 N sodium hydroxide, the reaction mixture was centrifuged to remove insoluble materials. Sodium sulfate was added to the supernatant at a final concentration of 0.18 g/ml. The precipitates were dissolved in 50 mM Tris-HCl (pH 8.0)/150 mM sodium chloride. The solution was passed through Sephadex G-150 (manufactured by Pharmacia Fine Chemicals Inc.) previously equilibrated with the same buffer, and F(ab')$_2$-containing fractions were collected. It was confirmed by the same method shown in 3-(1) that this F(ab')$_2$ could bind with human tryptase-like protein like the anti-human tryptase-like protein IgG.

[Test Examples]

Test on inhibition of HIV infection

1. Cells used

Human acute lymphoblastic leukemia cells, i.e., MOLT-4 cells (ATCC Number: CRL-1582), and CEM/LAV-1 cells obtained by persistent infection of CCRF-CEM cells (ATCC Number: CCL-119) with LAV-1, which is one of HIV [Barre-Sinoussi et al., Science, 220 , 868 (1983)], were used.

2. Incubation of cells

The cells were cultured in RPMI 1640 medium supplemented with 10% FCS under conditions of $37^{\circ}$C, 5% $CO_2$ and a relative humidity of 95%.

3. Test on inhibition of the syncytia formation

(1) Method

Test on inhibition of syncytia formation was performed according to the method of Lifson et al. [Lifson et al., J. Exp. Med., 164 , 2101 (1986)]. Briefly, a part of the culture of MOLT-4 cells was centrifuged at 2,000 rpm for 5 minutes to collect the cells. After removing the supernatant, the cells were suspended in ASF 104 medium (manufactured by Ajinomoto Co., Ltd.) to prepare a cell suspension ($5 \times 10^5$ cells/ml). To this suspension were added anti-human tryptase-like protein IgG, or its $F(ab')_2$ fragment at various concentrations. Each suspension was dispensed into a 96-well microtiter plate ($1 \times 10^5$ cells/well) and allowed to stand at $37^{\circ}$C for 30 minutes. The antibody had been diluted previously with ASF 104 medium (manufactured by Ajinomoto Co., Ltd.) followed by dialysis against the same medium at $4^{\circ}$C overnight. Two microliter of CEM/LAV-1 cells ($1 \times 10^7$ cells/ml) was added to each well and then thoroughly mixed. After culturing at $37^{\circ}$C for 12 hours in the presence of 5% $CO_2$, syncytia formation was examined under an inverted microscope (magnification x 200). More than 60% inhibition of syncytia formation was evaluated as + +; more than 30 to 60% inhibition was evaluated as +; and 30% or less inhibition was evaluated as ±; and no syncytia formation was evaluated as -. Thus, the inhibitory activity of syncytia formation was evaluated.

(2) Results

The results of the evaluation for the ability to inhibit the syncytia formation are shown in the following table.

| Inhibitory Activity of Anti-human tryptase-like protein IgG on Syncytia Formation | | | |
|---|---|---|---|
| Dilution fold of anti-human tryptase-like protein IgG | $1/3^0$ | $1/3^1$ | $1/3^2$ |
| Inhibition of syncytia formation | + + | + + | - |

As is clear from the above, the anti-human tryptase-like protein IgG significantly inhibits the syncytia formation, depending on its concentration. In the case of $F(ab')_2$ fragment of the anti-human tryptase-like protein IgG, inhibition of the syncytia formation was also observed to an extent similar to the case with the anti-human tryptase-like protein IgG. However, IgG purified from the non-immunized rabit sera did not inhibit the syncytia formation.

Example 3 Injections of anti-human tryptase-like protein antibody:

1. Preparation of affinity column

To 1 g of activated CH-Sepharose 4B (manufactured by Pharmacia Fine Chemicals Inc.) was added 1 mM HCl to swell the gel. The gel was then washed with 1 mM HCl. The purified human tryptase-like protein (200 μg) (0.1 M NaHCO₃ (pH 8.0) solution) was mixed with the swollen gel at room temperature for an hour. The gel was washed with 50 mM Tris-HCl (pH 8.0)/0.5 M sodium chloride, then with 50 mM sodium acetate (pH 4.0)/0.5 M sodium chloride. The washing procedure was repeated 3 times.

2. Purification of anti-human tryptase-like protein IgG using affinity column

The anti-human tryptase-like protein IgG prepared in Example 2 (2) was applied to the affinity column prepared in 1. above. After washing, the anti-human tryptase-like protein IgG bound to the column was eluted with 0.1 M phosphate buffer (pH 10.8). After pH was immediately adjusted to 7.5, the eluate was concentrated by ultrafiltration and dialyzed. The IgG was lyophilized and then dissolved in sterile water to prepare 16% solution.

The activity of the thus purified anti-human tryptase-like protein IgG for inhibiting the syncytia formation was examined by the method shown in Test Example. The activity was detected.

3. Preparation of injections of anti-human tryptase-like protein antibody

Using the anti-human tryptase-like protein IgG purified in 2. above, injections were prepared in a conventional manner in which glycine was used as a stabilizer and methyl thiolate was used as an antiseptic.

The injections may be intramuscularly administered in a dose of from 20 to 2,000 mg/kg body weight/day, preferably 50 to 1,000 mg/kg body weight/day, as IgG or its fragment.

Example 4 Preparation of anti-AIDS vaccine

The purified human tryptase-like protein was dialyzed against water and then lyophilized to prepare vaccines for AIDS.

The preparations are dissolved in physiological saline upon use and the solution is subcutaneously injected. The dose may be in the range of from 0.2 to 20 μg/kg-body weight, preferably 1 to 10 μg/kg body weight, per administration, as human tryptase-like protein.

The solution was administered to rabbit by the method shown in Example 2 (1) and evaluated by the method shown in Example 2 (3). Production of the antibody was confirmed.

**Claims**

1. A human tryptase-like protein capable of specifically binding with anti-rat tryptase antibody and comprising two subunits of about 35±3 KDa and four subunits of about 30±2 KDa, which are obtained from human T cells.

2. A human tryptase-like protein according to claim 1, which has a trypsin-like activity and a chymotrypsin-like activity.

3. A human tryptase-like protein according to claim 1 or 2, which is obtained from human acute lymphoblastic leukemia cell line, Molt 4.

4. Subunits of about 35±3 KDa or subunits of about 30±2 KDa of a human tryptase-like protein according to claim 1.

5. An anti-human tryptase-like protein antibody capable of specifically binding with a human tryptase-like protein or a fragment containing its antigen binding site.

6. A composition for the treatment of acquired immunodeficiency syndrome comprising as an effective ingredient an anti-human tryptase-like protein antibody or a fragment containing its antigen binding site,

EP 0 419 292 A1

together with a pharmaceutically acceptable carrier.

7. A vaccine for acquired immunodeficiency syndrome comprising a human tryptase-like protein according to claim 1.

12

# F I G. I

AGAROSE GEL

—

198K —

+

# F I G. 2

SDS – PAGE          WESTERN BLOT

75K —

50K —

39K —

27K —

17K —

F I G. 3

TRYPSIN—LIKE ACTIVITY

CHYMOTRYPSIN—LIKE ACTIVITY

ACTIVITY ($\mu$V/m$\ell$)

10

5

6.5  7.0  7.5  8.0  8.5  9.0  9.5

PH

EP 0 419 292 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF IMMUNOLOGY, vol. 134, no. 1, January 1985, pages 526-531, The American Association of Immunologists, US; L.B. SCHWARTZ: "Monoclonal antibodies against human mast cell tryptase demonstrate shared antigenic sites on subunits of tryptase and selective localization of the enzyme to mast cells" <br> * Entire document * | 3-6 | C 12 N 9/76 <br> C 12 P 21/08 <br> A 61 K 39/395 <br> A 61 K 37/547 |
| X | FEBS LETTERS, vol. 248, nos. 1,2, May 1989, pages 48-52, Elsevier Publishers B.V. (Biomedical Division); T. HATTORI et al: "Involvement of tryptase-related cellular protease(s) in human immunodeficiency virus type 1 infection" <br> * Entire document * | 4-6 | |
| P,X | EP-A-0 379 295 (TAISHO PHARM. CO.) <br> * Entire document * | 4-6 | |
| | | | TECHNICAL FIELDS SEARCHED (Int. Cl.5) |
| | | | C 12 N <br> C 12 P <br> A 61 K |

The present search report has been drawn up for all claims

| Place of search | Date of completion of search | Examiner |
|---|---|---|
| The Hague | 11 December 90 | GROENENDIJK M.S.M. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document